# EUROPÄISCHE PATENTSCHRIFT

(11) **EP 1 059 888 B1**
(45) Veröffentlichungstag und Bekanntmachung des Hinweises auf die Patenterteilung: **10.12.2003**
(21) Anmeldenummer: 98903983.9
(22) Anmeldetag: 05.03.1998
(51) Int. Cl.: A61B 17/74

(54) **MARKNAGEL MIT VERRIEGELUNGSLOCH**
INTRAMEDULLARY NAIL WITH LOCKING HOLE
CLOU D'OSTEOSYNTHESE INTRAMEDULLAIRE A TROU DE VERROUILLAGE

(43) Veröffentlichungstag der Anmeldung: 20.12.2000
(73) Patentinhaber: Synthes AG, Chur, 7002 Chur (CH); SYNTHES (U.S.A.), Paoli, PA 19301-1222 (US)
(72) Erfinder: ACKERET, Roman, CH-4310 Rheinfelden (CH); SENN, Peter, CH-4437 Waldenburg (CH); HUNGERBÜHLER, Ruth, CH-4438 Langenbruck (CH); GRASS, René, D-01326 Dresden (DE)
(74) Vertreter: Lusuardi, Werther Giovanni, Dr.
(86) Internationale Anmeldenummer: CH9800087
(87) Internationale Veröffentlichungsnummer: WO99044528

(56) Entgegenhaltungen:
- EP-A- 0 411 273
- WO-A-98/30164
- DE-A- 3 730 570
- US-A- 3 433 220
- US-A- 4 103 683

## Beschreibung

Die Erfindung bezieht sich auf einen Marknagel mit Verriegelungsloch gemäss dem Oberbegriff des Patentanspruchs 1.

Oft ist im Falle von Femurfrakturen eine rasche Mobilisation erforderlich. In solchen Fällen oder auch wenn das Kniegelenk schon offen ist, kann ein Marknagel von distal, das heisst vom Knie her in den Oberschenkel implantiert werden. Dabei werden solche Marknägel vielfach mit einer Spiralklinge, die lateral medial eingeführt wird, zur Fixation des Marknagels implantiert. Im Marknagel ist zu diesem Zweck eine Bohrung mit einem länglichen Querschnitt, zum Beispiel in der Form eines Schlitzes oder in einer zu einem Schlüsselloch ähnlichen Form angebracht. Anstelle der Spiralklinge kann der Marknagel auch mit Verriegelungsschrauben, die ebenfalls lateral medial eingeführt werden, so verriegelt werden, dass sich der Marknagel in Richtung seiner Längsachse nicht verschieben kann. Üblicherweise werden heute die Öffnungen für die Verriegelungsmittel so gestaltet, dass der Marknagel entweder nur mit Verriegelungsschrauben oder nur mit einer Spiralklinge axial fixiert werden kann. Auf diese Weise entsteht ein grosses Sortiment von Marknägeln, die jeweils zusammen mit einem bestimmten Verriegelungsmittel implantiert werden müssen.

Ein Marknagel mit einem zur Längsachse des Marknagels abgewinkelten Nagelkopf und einem Verriegelungsloch quer zur Achse des Nagelkopfes ist in der US 3,433,220 ZICKEL offenbart. Das Verriegelungsloch dieses bekannten Marknagels umfasst ein quer zur Längsachse des Nagelkopfes verlaufendes Langloch, eine winklig zur Längsachse des Nagelkopfes verlaufende Bohrung, deren Achse in der durch die Längsachse des Marknagels und die Längsachse des Nagelkopfes gebildeten Ebene liegt, und eine konzentrisch zur Längsachse des Nagelkopfes verlaufende zweite Bohrung mit einem Innengewinde am Nagelkopfende. Diese zweite Bohrung dient zur Aufnahme einer Feststellschraube, mittels welcher ein quer zum Nagelkopf eingeführtes Verriegelungsmittel im Nagelkopf fixierbar ist. Die Anordnung dieser Feststellschraube eignet sich jedoch nur zur Fixation von zylindrischen oder prismatischen Verriegelungsmitteln, während Spiralklingen axial nicht gesichert werden können.

Hier will die Erfindung Abhilfe schaffen. Der Erfindung liegt die Aufgabe zugrunde, einen Marknagel mit einem den Marknagel quer durchdringenden Verriegelungsloch zu versehen, das gestattet, je nach Bedarf das geeignete Verriegelungsmittel zu verwenden. Die Form des Verriegelungsloches ist so gestaltet, dass eine Spiralklinge oder mehrere Verriegelungsschrauben lateral medial den Marknagel durchdringend implantiert werden können. Spiralklinge oder Verriegelungsschrauben erfüllen durch die spezielle Gestaltung des Verriegelungsloches im Marknagel die Aufgabe, dass nach deren Implantation der Marknagel in Richtung seiner Längsachse nicht mehr verschiebbar ist

Die Erfindung löst die gestellte Aufgabe mit einem Marknagel mit Verriegelungsloch, welcher die Merkmale des Anspruchs 1 aufweist.

In einer Ausführungsform des erfindungsgemässen Marknagels mit Verriegelungsloch verläuft dieses Verriegelungsloch quer zur Längsachse des Marknagels und durchdringt den Marknagel. Das Verriegelungsloch umfasst eine quer zur Längsachse des Marknagels verlaufende, den Marknagel durchdringende Durchgangsöffnung, eine die Durchgangsöffnung nicht berührende ebenfalls quer zur Längsachse des Marknagels verlaufende und den Marknagel durchdringende Bohrung und einen die Durchgangsöffnung und die Bohrung verbindenden, zur Durchgangsöffnung nicht parallelen Schlitz. Der Schlitz verbindet die Durchgangsöffnung und die Bohrung in Richtung der Längsachse des Marknagels auf ihrer gesamten dem Durchmesser des Marknagels entsprechenden Länge und durchdringt den Marknagel quer zur Längsachse des Marknagels. Das Verriegelungsloch ist derart gestaltet, dass eine Spiralklinge unter Ausführung einer schraubenförmigen Bewegung von lateral nach medial durch das Verriegelungsloch hindurchgeführt werden kann. Dabei ist die Form des Verriegelungsloches so beschaffen, dass anstelle der Spiralklinge auch zwei Schrauben lateral medial den Marknagel durchdringend implantiert werden können. Die Schrauben und die Sprialklinge verlaufen bezüglich ihrer Längsachsen parallel in einer die Längsachse des Marknagels enthaltenden Ebene. Sowohl die Schrauben als auch die Spiralklinge durchdringen den Marknagel diametral. Im Falle dass Schrauben implantiert werden, muss eine davon, um eine Verschiebung des Marknagels in axialer Richtung zu verhindern, durch die Bohrung verlaufend montiert werden. Die Andere kann in der Durchgangsöffnung positioniert werden. Wird die Schraube in der Durchgangsöffnung an den vom Schlitz entfernten Rand der Durchgangsöffnung gesetzt, so hilft sie zusätzlich, eine Verschiebung in Längsrichtung des Marknagels zu verhindern.

Eine weitere Ausführungsform des erfindungsgemässen Marknagels mit Verriegelungsloch unterscheidet sich von der oben beschriebenen Ausführungsform nur dadurch, dass der Schlitz, welcher die Durchgangsöffnung und die Bohrung quer durch den Marknagel durchgehend verbindet, unter einem Winkel zur Längsachse des Marknagels verläuft.

Wiederum eine weitere Ausführungsform des erfindungsgemässen Marknagels mit Verriegelungsloch unterscheidet sich von der zuerst beschriebenen Variante nur dadurch, dass der Schlitz eine parallele Schnittebene aufweist, die die Längsachse des Marknagels und eine zweite gegenüber der Zentralachse der Bohrung einen Winkel einschliessende Gerade enthält. Das heisst, wenn die Längsachse der Durchgangsöffnung und die Längsachse der Bohrung parallel sind und in einer die Längsachse des Marknagels enthaltenen Ebene liegen, verläuft der die Durchgangsöffnung und die Bohrung verbindende Schlitz unter einem Winkel zu den erwähnten Längsachsen der Bohrung und der Durchgangsöffnung diametral durch den Marknagel.

Eine andere Ausführungsform des erfindungsgemässen Marknagels mit Verriegelungsloch unterscheidet sich von den vorangehend beschriebenen Varianten nur dadurch, dass der Schlitz parallel zu einer Ebene, die die Längsachse des Marknagels und eine zweite gegenüber der Zentralachse der Bohrung einen Winkel von 15° einschliessende Gerade enthält, verläuft.

Eine weitere Ausf ührungsform des erfindungsgemässen Marknagels mit Verriegelungsloch unterscheidet sich von den vorangehend beschriebenen Varianten nur dadurch, dass die zum erfindungsgemässen Verriegelungsloch gehörende Durchgangsöffnung und die ebenfalls zum erfindungsgemässen Verriegelungsloch gehörende Bohrung rechtwinklig zur Längsachse des Marknagels verlaufen.

Wiederum eine weitere Ausführungsform des erfindungsgemässen Marknagels mit Verriegelungsloch ist dadurch von den vorangehend beschriebenen Varianten verschieden, dass die Durchgangsöffnung ein Langloch mit einer Breite b und einer Länge a ist.

Wiederum eine andere Ausführungsform des erfindungsgemässen Marknagels mit Verriegelungsloch unterscheidet sich von der vorangehend beschriebenen Variante nur dadurch, dass die Breite b des zum erfindungsgemässen Verriegelungsloch zählenden Langloches dem Durchmesser der ebenfalls zum erfindungsgemässen Verriegelungsloch zählenden Bohrung entspricht.

Eine andere Ausführungsform des erfindungsgemässen Marknagels mit Verriegelungsloch zeichnet sich gegenüber den vorangehend beschriebenen Varianten dadurch aus, dass die zum erfindungsgemässen Verriegelungsloch gehörende Durchgangsöffnung als Querschnitt anstelle eines Langloches einen ovalen Querschnitt mit der grossen Halbachse a/2 und der kleinen Halbachse b/2 aufweist.

Wieder eine andere Ausführungsform des erfindungsgemässen Marknagels mit Verriegelungsloch zeichnet sich gegenüber den vorangehend beschriebenen Varianten dadurch aus, dass die zum erfindungsgemässen Verriegelungsloch zählende Durchgangsöffnung einen kreisförmigen Querschnitt hat.

Eine weitere Ausführungsform des erfindungsgemässen Marknagels mit Verriegelungsloch besteht darin, dass der Schlitz in einem Schnitt rechtwinklig zur Längsachse des Marknagels eine Breite c aufweist, die zwischen 10 % und 90 % der Breite b des Langloches beträgt.

Eine weitere Ausführungsform des erfindungsgemässen Marknagels mit Verriegelungsloch besteht darin, dass der Schlitz in einem Schnitt rechtwinklig zur Längsachse des Marknagels eine Breite c aufweist, die zwischen 30 % und 50 % der Breite b des Langloches beträgt.

Die durch die Erfindung erreichten Vorteile sind im wesentlichen darin zu sehen, dass dank des erfindungsgemässen Verriegelungsloches für Verriegelungsmittel in Marknägeln für zwei verschiedene Verriegelungsarten derselbe Marknagel eingesetzt werden kann. Die Form des Verriegelungsloches ist so gestaltet, dass sowohl die Spiralklinge als auch die Verriegelungsschrauben innerhalb des erfindungsgemässen Verriegelungsloches so plaziert sind, dass der Marknagel in Richtung seiner Längsachse nicht mehr verschoben werden kann. Die Neuheit des erfindungsgemassen Verriegelungsloches liegt darin, dass ein Marknagel mit zwei verschiedenen Verriegelungsmitteln fixiert werden kann. Der Vorteil für den Anwender und den Produzenten liegt im kleineren Sortiment. Die Entscheidung, welche Verriegelung gewählt werden soll, kann somit sehr kurzfristig getroffen werden.

Die Erfindung und Weiterbildungen der Erfindung werden im folgenden anhand der teilweise schematischen Darstellungen mehrerer Ausführungsbeispiele noch näher erläutert.

Es zeigen:
Fig. 1 einen Aufriss eines Teils einer Ausführungsform des erfindungsgemässen Marknagels mit dem Verriegelungsloch sowie einen Querschnitt durch den Marknagel senkrecht zur Längsachse;
Fig. 2 einen Aufriss sowie einen Seitenriss eines Teils einer Ausführungsform des erfindungsgemässen Marknagels mit dem Verriegelungsloch und darin eingeführter Spiralklinge;
Fig. 3 einen Aufriss sowie einen Seitenriss eines Teils einer Ausführungsform des erfindungsgemässen Marknagels mit dem Verriegelungsloch und darin eingeführten Verriegelungsschrauben;
Fig. 4 einen Aufriss eines Teils des Marknagels mit einer Variante des Verriegelungsloches sowie einen Querschnitt durch den Marknagel senkrecht zur Längsachse; und
Fig. 5 einen Aufriss eines Teils des Marknagels mit einer anderen Variante des Verriegelungsloches sowie einen Querschnitt durch den Marknagel senkrecht zur Längsachse.

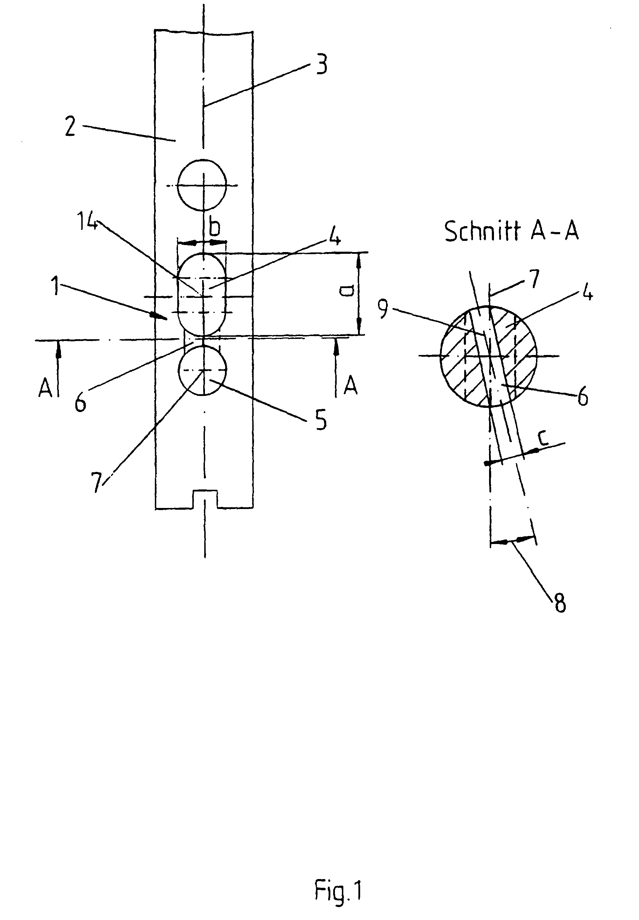

Die in Fig.1 dargestellte Ausführungsform des erfindungsgemässen Marknagels 2 mit Verriegelungsloch 1 für verschiedene Verriegelungsmittel in Marknägeln 2 zeigt einen Ausschnitt aus dem Marknagel 2 mit dem Verriegelungsloch 1 im Aufriss sowie einen Querschnitt des Marknagels 2. Das Verriegelungsloch 1 verläuft quer zur Längsachse 3 des Marknagels 2 und durchdringt den Marknagel 2 diametral. Das Verriegelungsloch 1 umfasst eine quer zur Längsachse 3 des Marknagels 2 diametral verlaufende Durchgangsöffnung 4, beispielsweise ein Langloch, eine die Durchgangsöffnung 4 nicht berührende ebenfalls quer zur Längsachse 3 des Marknagels 2 und parallel zur Durchgangsöffnung 4 verlaufende Bohrung 5 mit einer Zentralachse 7 und einen Schlitz 6. Der Schlitz 6 verbindet die Durchgangsöffnung 4 und die Bohrung 5 in Richtung der Längsachse 3 des Marknagels 2 auf ihrer gesamten dem Durchmesser des Marknagels 2 entsprechenden Länge und durchdringt den Marknagel 2 quer zur Längsachse 3 des Marknagels 2. Der Schlitz 6 verläuft in einem Querschnitt des Marknagels 2 nicht parallel zur Durchgangsöffnung 4, durchdringt jedoch den Marknagel 2 diametral. Im weiteren weist der Schlitz 6 eine parallele Schnittebene auf, die die Längsachse 3 des Marknagels 2 und eine zweite gegenüber der Zentralachse 7 der Bohrung 5 einen Winkel 8 einschliessende Gerade 9 enthält. In der hier dargestellten Ausführungsform des erfindungsgemässen Marknagels 2 mit Verriegelungsloch 1 beträgt der Winkel zwischen der Zentralachse 7 der Bohrung 5 und einer zum Schlitz 6 parallelen Geraden 9 einen Winkel von 15°. Die zum Verriegelungsloch 1 gehörende Durchgangsöffnung 4 und die ebenfalls zum Verriegelungsloch 1 gehörende Bohrung 5 verlaufen quer zur Längsachse 3 des Marknagels 2. Die Durchgangsöffnung 4 ist als Langloch ausgebildet und hat eine Länge a und eine Breite b, die dem Durchmesser der ebenfalls zum Verriegelungsloch 1 zählenden Bohrung 5 entspricht. Der Querschnitt der Durchgangsöffnung 4 kann anstelle eines Langloches 4 auch oval oder rund sein.

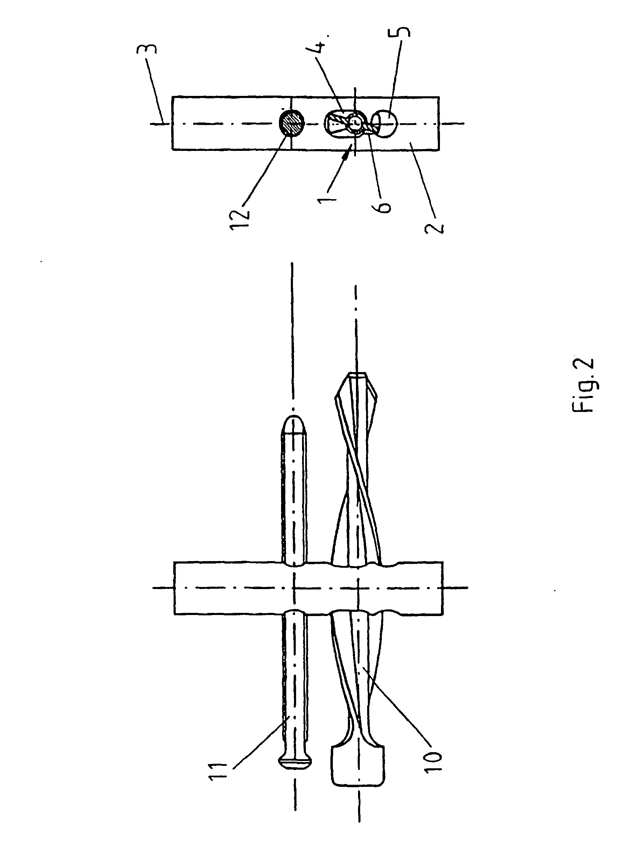

Fig. 2 zeigt einen Teil eines Marknagels 2 mit dem Verriegelungsloch 1, das dem oben Beschriebenen entspricht. Zusätzlich zu dem in Fig. 1 dargestellten Marknagel 2 sind hier eine in das Verriegelungsloch 1 des Marknagels 2 eingeführte Spiralklinge 10 und eine durch eine weitere Bohrung 12 im Marknagel 2 eingeführte Verriegelungsschraube 11 dargestellt.

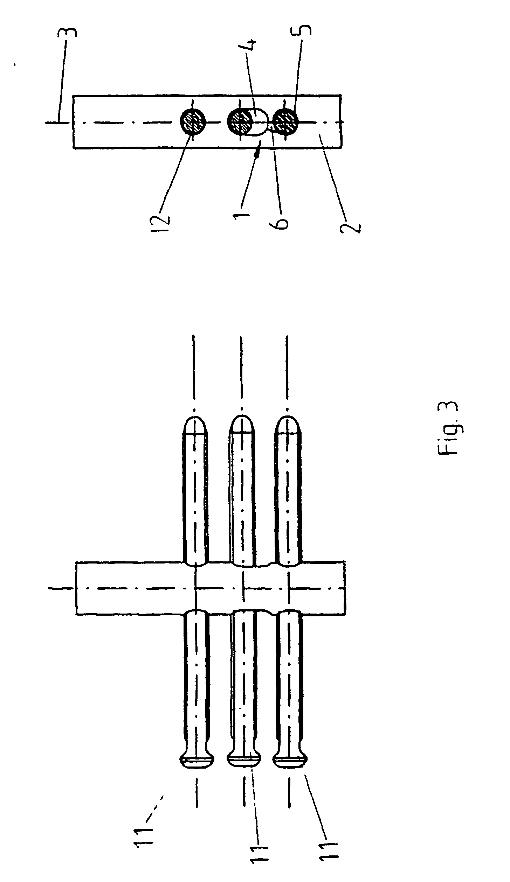

In Fig. 3 ist ein Teil eines Marknagels 2 mit der unter Fig. 1 beschriebenen Ausführungsform des Verriegelungslochs 1 dargestellt. Zusätzlich zu dem in Fig. 1 gezeigten Marknagel 2 sind in Fig. 3 zwei durch das Verriegelungsloch 1 des Marknagels 2 eingeführte Verriegelungsschrauben 11 und eine weitere durch die Bohrung 12 im Marknagel 2 eingeführte Verriegelungsschraube 11 dargestellt.

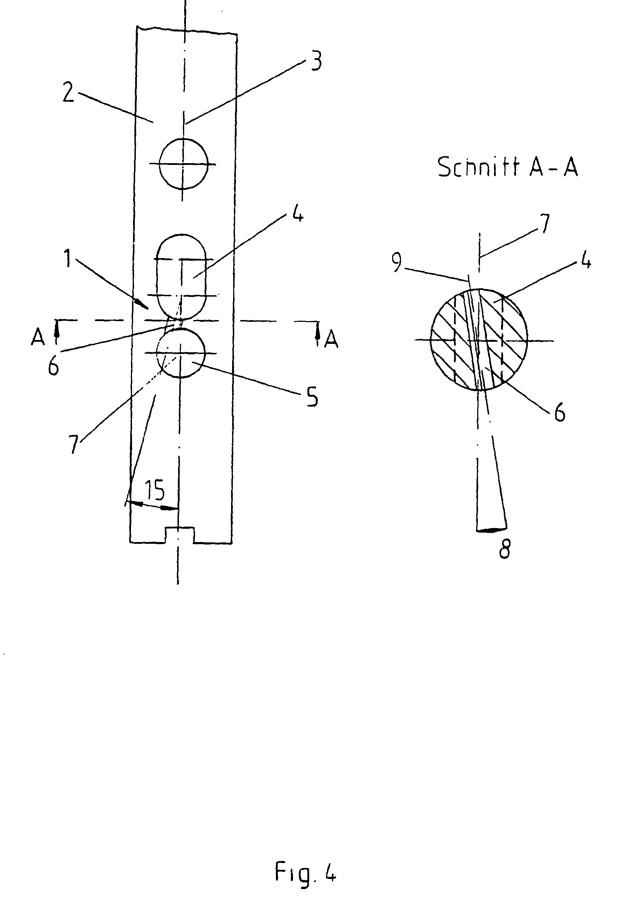

Fig. 4 zeigt eine Ausführungsform des erfindungsgemässen Marknagels 2 mit Verriegelungsloch 1, die sich von der in Fig. 1 dargestellten Ausführungsform darin unterscheidet, dass der Schlitz 6, welcher die als Langloch ausgebildete Durchgangsöffnung 4 und die Bohrung 5 in Richtung der Längsachse 3 des Marknagels 2 verbindet, zwischen dem Langloch 4 und der Bohrung 5 unter einem Winkel 15 gegenüber der Längsachse 3 des Marknagels 2 verläuft. Der Schlitz 6 verläuft auch bei dieser Variante diametral und schliesst einen Winkel 8 zur Zentralachse 7 der Bohrung 5 ein.

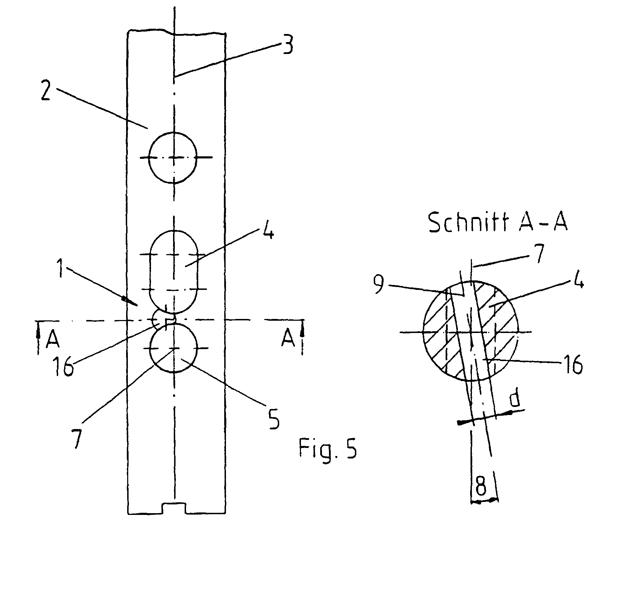

Die in Fig. 5 dargestellte Ausführungsform des erfindungsgemässen Marknagels 2 mit Verriegelungsloch 1 enthält wie die vorangehend beschriebenen Ausführungsformen eine als Langloch ausgebildete Durchgangsöffnung 4, welche den Marknagel 2 diametral durchdringt, eine den Marknagel 2 diametral und parallel zur Durchgangsöffnung 4 durchdringende Bohrung 5 mit der Zentralachse 7 und einen gebohrten Schlitz 16. Der Schlitz 16 besteht aus einer Bohrung mit dem Durchmesser d, wobei die Zentralachse 9 der Bohrung 16 mit der Zentralachse 7 der Bohrung 5 einen Winkel 8 einschliesst.

## Patentansprüche

1. Marknagel (2) mit Verriegelungsloch (1), wobei dieses Verriegelungsloch (1) den Marknagel (2) quer zur Längsachse (3) des Marknagels (2) durchdringt und
A) eine den Marknagel (2) quer zur Längsachse (3) durchdringende zylindrische Durchgangsöffnung (4); und
B) eine die Durchgangsöffnung (4) nicht berührende ebenfalls den Marknagel (2) quer zur Längsachse (3) durchdringende Bohrung (5), welche gegenüber der Durchgangsöffnung (4) in Richtung der Längsachse (3) versetzt ist, umfasst;
**dadurch gekennzeichnet, dass**
C) das Verriegelungsloch (1) einen Schlitz (6) beinhaltet, welcher die Durchgangsöffnung (4) und die Bohrung (5) durchgehend verbindet; und
D) der Schlitz (6) in einem rechtwinklig zur Längsachse (3) des Marknagels (2) verlaufenden Querschnitt des Marknagels (2) nicht parallel zur Durchgangsöffnung (4) verläuft.

2. Marknagel (2) mit Verriegelungsloch (1) nach Anspruch 1, **dadurch gekennzeichnet, dass** die Durchgangsöffnung eine Breite b und eine Länge a hat und sich die Länge a parallel oder mit einem Winkel zur Längsachse (3) des Marknagels (2) erstreckt.

3. Marknagel (2) mit Verriegelungsloch (1) nach Anspruch 1 oder 2, **dadurch gekennzeichnet, dass** der Schlitz (6) die Durchgangsöffnung (4) und die Bohrung (5) diametral verbindet.

4. Marknagel (2) mit Verriegelungsloch (1) nach einem der Ansprüche 1 bis 3, **dadurch gekennzeichnet, dass** der Schlitz (6) eine parallele sich in Richtung der Längsachse (3) erstreckende Schnittebene aufweist, die die Längsachse (3) des Marknagels (2) und eine zweite gegenüber der Zentralachse (7) der Bohrung (5) einen Winkel (8) einschliessende Gerade (9) enthält.

5. Marknagel (2) mit Verriegelungsloch (1) nach Anspruch 4, **dadurch gekennzeichnet, dass** der Schlitz (6) parallel zu einer Ebene, die die Längsachse (3) des Marknagels (2) und eine zweite gegenüber der Zentralachse (7) der Bohrung (5) einen Winkel (8) mit einem Betrag von zwischen 5° und 25° einschliessende Gerade (9) enthält, verläuft.

6. Marknagel (2) mit Verriegelungsloch (1) nach einem der Ansprüche 1 bis 5, **dadurch gekennzeichnet, dass** die Durchgangsöffnung (4) und die Bohrung (5) zueinander parallel und diametral zum Marknagel (2) verlaufen.

7. Marknagel (2) mit Verriegelungsloch (1) nach einem der Ansprüche 1 bis 6, **dadurch gekennzeichnet, dass** die Durchgangsöffnung (4) und die Bohrung (5) senkrecht zur Längsachse (3) des Marknagels (2) verlaufen.

8. Marknagel (2) mit Verriegelungsloch (1) nach einem der Ansprüche 1 bis 7, **dadurch gekennzeichnet, dass** die Durchgangsöffnung (4) ein Langloch mit der Länge a und der Breite b ist.

9. Marknagel (2) mit Verriegelungsloch (1) nach Anspruch 8, **dadurch gekennzeichnet, dass** die Breite b der Durchgangsöffnung (4) dem Durchmesser der Bohrung (5) entspricht.

10. Marknagel (2) mit Verriegelungsloch (1) nach Anspruch 8 oder 9, **dadurch gekennzeichnet, dass** der Schlitz (6) in einem Schnitt rechtwinklig zur Längsachse (3) des Marknagels (2) eine Breite c aufweist, die zwischen 10 % und 90 % der Breite b der Durchgangsöffnung (4) beträgt.

11. Marknagel (2) mit Verriegelungsloch (1) nach einem der Ansprüche 8 bis 10, **dadurch gekennzeichnet, dass** der Schlitz (6) in einem Schnitt rechtwinklig zur Längsachse (3) des Marknagels (2) eine Breite c aufweist, die zwischen 30 % und 50 % der Breite b der Durchgangsöffnung (4) beträgt.

12. Marknagel (2) mit Verriegelungsloch (1) nach einem der Ansprüche 8 bis 11, **dadurch gekennzeichnet, dass** das Verhältnis von Länge a zu Breite b der Durchgangsöffnung (4) in einem Bereich von 1,1 bis 2,5 liegt.

13. Marknagel (2) mit Verriegelungsloch (1) nach einem der Ansprüche 2 bis 7, **dadurch gekennzeichnet, dass** die Durchgangsöffnung (4) den Querschnitt eines Ovals mit den Halbachsen a/2 und b/2 aufweist.

14. Marknagel (2) mit Verriegelungsloch (1) nach Anspruch 1, **dadurch gekennzeichnet, dass** die Durchgangsöffnung (4) einen runden Querschnitt aufweist.

## Claims

1. A medullar pin (2) fitted with a locking opening (1) which crosses the pin (2) transversely to its longitudinal axis (3), and
(A) comprising a cylindrical passage (4) penetrating the medullar pin (2) transversely to the longitudinal axis (3) of the pin (2), and
(B) a borehole (5) not touching the passage (4) and also penetrating the medullar pin (2) transversely to the longitudinal axis (3) and offset from the passage (4) toward the longitudinal axis (3),
**characterized in that**
(C) the locking opening (1) contains a slot (6) continuously connecting the passage (4) and the borehole (5), and
(D) the slot (6) is skewed to the passage (4) in a cross-section of this pin (2) that runs perpendicularly to the longitudinal axis (3) of the pin (2).

2. Medullary pin (2) fitted with a locking opening (1) as claimed in claim 1, **characterized in that** the passage is of width b and length a and **in that** the length a runs parallel or at an angle to the longitudinal axis (3) of the pin (2).

3. Medullar pin (2) fitted with a locking opening (1) as claimed in either of claims 1 and 2, **characterized in that** the slot (6) diametrically connects the passage (4) and the borehole (5).

4. Medullar pin (2) fitted with a locking opening as claimed in one of claims 1 through 3, **characterized in that** the slot (6) comprises a sectional plane parallel to the direction of the longitudinal axis (3) of the pin (2), said plane containing this axis (3) and furthermore a second straight line (9) subtending an angle (8) with the central axis (7) of the borehole (5).

5. Meduiiar pin (2) fitted with a locking opening (1) as claimed in claim 4, **characterized in that** the slot (6) runs parallel to a plane containing the longitudinal axis (3) of the pin (2) and a second straight line (9) which together with the central axis (7) of the borehole (5) subtends an angle between 5 and 25°.

6. Medullar pin fitted with a locking opening (1) as claimed in one of claims 1 through 5, **characterized in that** the passage (4) and the borehole (5) are mutually parallel and diametrical with respect to the pin (2).

7. Medullar pin (2) fitted with a locking opening (1) as claimed in one of claims 1 through 6, **characterized in that** the passage (4) and the borehole (5) run perpendicularly to the longitudinal axis (3) of the pin (2).

8. Medullar pin (2) fitted with a locking opening (1) as claimed in one of claims 1 through 7, **characterized in that** the passage (4) is an elongated slot of length a and width b.

9. Medullar pin (2) fitted with a locking opening (1) as claimed in claim 8, **characterized in that** the width b of the passage (4) corresponds to the diameter of the borehole (5).

10. Medullar pin (2) fitted with a locking opening (1) as claimed in claim 8 or 9, **characterized in that** the slot (6) when viewed in a cross-section perpendicular to the longitudinal axis (3) of the pin (2) is of width c amounting to 10 to 90 % of the width b of the passage (4).

11. Medullar pin (2) fitted with a locking opening (1) as claimed in one of claims 8 through 10, **characterized in that** the slot (6) when seen in a section perpendicular to the longitudinal axis (3) of the pin (2) is of width c amounting to 30 to 50 % of the width b of the passage (4).

12. Medullar pin (2) fitted with a locking opening (1) as claimed in one of claims 8 through 11, **characterized in that** the ratio of the length a to the width b of the passage (4) is in the range of 1.1 to 2.5.

13. Medullar pin (2) fitted with a locking opening (1) as claimed in one of claims 2 through 7, **characterized in that** the cross-section of the passage (4) is elliptic with semi-axes a/2 and b/2.

14. Medullar pin (2) fitted with a locking opening (1) as claimed in claim 1, **characterized in that** the cross-section of the passage (4) is circular.

## Revendications

1. Clou médullaire (2) doté d'un trou de verrouillage (1), ce trou de verrouillage (1) traversant le clou médullaire (2) transversalement par rapport à l'axe longitudinal (3) du clou médullaire (2) et
A) une ouverture cylindrique de passage (4) qui traverse le clou médullaire (2) transversalement par rapport à l'axe longitudinal (3) et
B) un alésage (5) qui n'est pas en contact avec l'ouverture de passage (4) et qui traverse également le clou médullaire (2) transversalement par rapport à son axe longitudinal (3) et qui est décalé dans la direction de l'axe longitudinal (3) par rapport à l'ouverture de passage (4),
**caractérisé en ce que**
C) le trou de verrouillage (1) contient une fente (6) qui relie de manière continue l'ouverture de passage (4) et l'alésage (5) et
D) la fente (6) s'étend dans une section transversale du clou médullaire (2) qui n'est pas parallèle à l'ouverture de passage (4), et qui est perpendiculaire à l'axe longitudinal (3) du clou médullaire (2).

2. Clou médullaire (2) doté d'un trou de verrouillage (1) selon la revendication 1, **caractérisé en ce que** l'ouverture de passage a une largeur b et une longueur a et **en ce que** la longueur a s'étend parallèlement ou obliquement par rapport à l'axe longitudinal (3) du clou médullaire (2).

3. Clou médullaire (2) doté d'un trou de verrouillage (1) selon les revendications 1 ou 2, **caractérisé en ce que** la fente (6) relie l'ouverture de passage (4) et l'alésage (5) dans le sens diamétral.

4. Clou médullaire (2) doté d'un trou de verrouillage (1) selon l'une des revendications 1 à 3, **caractérisé en ce que** la fente (6) présente un plan qui s'étend parallèlement à la direction de l'axe longitudinal (3) et qui contient l'axe longitudinal (3) du clou médullaire (2) et une deuxième droite (9) qui forme avec l'axe central (7) de l'alésage (5) un angle (8).

5. Clou médullaire (2) doté d'un trou de verrouillage (1) selon la revendication 4, **caractérisé en ce que** la fente (6) s'étend parallèlement à un plan qui contient l'axe longitudinal (3) du clou médullaire (2) et une deuxième droite qui forme avec l'axe central (7) de l'alésage (5) un angle (8) compris entre 5° et 25°.

6. Clou médullaire (2) doté d'un trou de verrouillage (1) selon l'une des revendications 1 à 5, **caractérisé en ce que** l'ouverture de passage (4) et l'alésage (5) s'étendent parallèlement l'un à l'autre et dans le sens diamétral par rapport au clou médullaire (2).

7. Clou médullaire (2) doté d'un trou de verrouillage (1) selon l'une des revendications 1 à 6, **caractérisé en ce que** l'ouverture de passage (4) et l'alésage (5) s'étendent perpendiculairement à l'axe longitudinal (3) du clou médullaire (2).

8. Clou médullaire (2) doté d'un trou de verrouillage (1) selon l'une des revendications 1 à 7, **caractérisé en ce que** l'ouverture de passage (4) est un trou oblong d'une longueur a et d'une largeur b.

9. Clou médullaire (2) doté d'un trou de verrouillage (1) selon la revendication 8, **caractérisé en ce que** la largeur b de l'ouverture de passage (4) correspond au diamètre de l'alésage (5).

10. Clou médullaire (2) doté d'un trou de verrouillage (1) selon les revendications 8 ou 9, **caractérisé en ce que** la fente (6) présente dans une coupe perpendiculaire à l'axe longitudinal (3) du clou médullaire (2) une largeur c qui vaut entre 10 % et 90 % de la largeur b de l'ouverture de passage (4).

11. Clou médullaire (2) doté d'un trou de verrouillage (1) selon l'une des revendications 8 à 10, **caractérisé en ce que** la fente (6) présente dans une coupe perpendiculaire à l'axe longitudinal (3) du clou médullaire (2) une largeur c qui vaut entre 30 % et 50 % de la largeur b de l'ouverture de passage (4).

12. Clou médullaire (2) doté d'un trou de verrouillage (1) selon l'une des revendications 8 à 11, **caractérisé en ce que** le rapport entre la longueur a et la largeur b de l'ouverture de passage (4) est compris dans la plage de 1,1 à 2,5.

13. Clou médullaire (2) doté d'un trou de verrouillage (1) selon l'une des revendications 2 à 7, **caractérisé en ce que** l'ouverture de passage (4) présente une section transversale ovale dont les demi-axes valent a/2 et b/2.

14. Clou médullaire (2) doté d'un trou de verrouillage (1) selon la revendication 1, **caractérisé en ce que** l'ouverture de passage (4) présente une section transversale circulaire.
